# EUROPEAN PATENT APPLICATION

(11) **EP 1 364 668 A1**
(43) Date of publication of application: **26.11.2003**
(21) Application number: 02712281.1
(22) Date of filing: 06.02.2002
(51) Int. Cl.: A61M 5/32

(54) **SAFE INJECTOR AND INJECTION NEEDLE FOR SAFE INJECTOR**

(30) Priority: 06.02.2001 JP 2001028960; 06.02.2002 JP 2002029082
(71) Applicant: JMS Co., Ltd., Hiroshima-shi, Hiroshima 730-8652 (JP)
(72) Inventor: KUNISHIGE, Takahiko, JMS Co., Ltd, Hiroshima-shi, Hiroshima 730-8652 (JP)
(74) Representative: Gassner, Wolfgang, Dr.
(86) International application number: JP0200954
(87) International publication number: WO02062411

(57) **Abstract**

A safety injector capable of preventing an erroneous needling by pulling the needle tube part of an injection needle into an outer tube after use, comprising a convergently formed tubular lure part and an annular projected part coaxially surrounding the lure part provided at the opening tip part of the outer tube, characterized in that an engagement projected part is formed on the tip side inner surface of the annular projected part; an injection needle for medical treatment used in the safety injector, comprising an injection needle, a needle mounting part fixed to the base part of the needle tube part of the injection needle, and a needle base covering the outside of the needle mounting part with the base side of the needle mounting part left uncovered and holding the needle mounting part by the inner cavity thereof in contact with each other so that the needle mounting part can be separated therefrom, characterized in that an engagement projection is formed on the base part peripheral edge of the needle base, whereby the problems on the needle and the needle base can be solved, and not only a dedicated injection needle but also a general injection needle can be used.

## Description

### Technical Field

The present invention relates to a safety injector, i.e., a safety syringe, and particularly to a safety syringe of a type of preventing inadvertent needling by retracting the needle into the outer cylinder after use, and an injection needle for the safety injector.

The present invention particularly relates to a safety injector and an injection needle for the safety injector which solves the problem of the detachment of the needle and needle base in the field of safety injectors and injection needles for the safety injectors, and allows the use of not only special purpose type needles but also general purpose type needles.

### Background Art

Various types of safety syringes have been devised for the purpose of preventing the operator from inadvertently injuring his/her finger upon covering the injection needle with a needle cover after needling. There are a wide variety of such safety syringes; among them are an outer-cylinder sheath system whereby the entire outer-cylinder including an injection needle is covered with a cover which is slidable on the outside of the outer-cylinder, and a needle retraction system whereby the needle is retracted into the outer-cylinder. Syringes (hereinafter, also referred to as an injector) of the latter system include the injector of which mechanism is described in Japanese Patent No. 2887643.

The injector disclosed in the above-mentioned patent is simple and useful; however, it had various problems in practical use.

The first problem relates to potential detachment of the needle and the needle base. If a needle mounting part is pressed by a plunger in a state that the needle base (which is disclosed as an outer collar member in the above mentioned patent) is not securely fitted to the opening front-end-part, the needle tube part and the mounting part for the needle tube part would become detached from the opening front-end-part along with the needle base, thereby posing a danger as well as a pollution problem.

Such loose fitting can occur during transportation or storage even if it has not occurred during production, and the frequency of the occurrence would vary depending on the transportation and storage conditions. A difficulty existed in that it was impossible to determine a loose fitting from appearance and therefore the detachment of the needle would occur in most cases without the operator's anticipation. For example, upon pushing the inner cylinder into the outer cylinder to retract the needle into the outer cylinder after use, there may occur a trouble that a bare (coverless) injection needle falls out.

In the above mentioned needle retractable safety syringe, in order to retract the needle tube part into the outer cylinder, it is necessary to engage the blind hole at the inner cylinder front-end-part with the protruding part at the lower-end-side of the needle mounting part and, to secure the engagement, the inner cylinder needs to be firmly pushed deep into the bottom (abutment point) of the internal cavity of the outer cylinder. Upon pushing, the injection needle may fall out along with the needle base from the injector by a pushing pressure of the inner cylinder if the needle base (outer collar member) is not securely fitted into the opening front-end-part of the injector. Such a fallout of the injection needle without operator's anticipation will pose not only a risk of inadvertent needling with a bare injection needle, but also a problem of contamination by the patient blood remained in the injector or the injection needle. In order to prevent such fallouts mentioned above, a total inspection (for checking the loosening of fitting) in the manufacturing process and a check-up at the operation site before use are needed; however, that was not practical for the matter of efficiency and time and there was a desire for improvement of such situation.

The second problem relates to the fact that such conventional safety syringes were unable to be mounted with (or to use) a general purpose type needle. A typical disposal injector utilizes fitting by a taper to mount the needle on the opening front-end-part of the injector. That is, a taper formed on the outer surface of the opening front-end-part of the injector and another taper formed on the internal cavity surface of the needle base are jointed to fit them together. By this taper fit, the liquid-tightness in the outer cylinder of the injector is maintained. However, the needle retractable injector disclosed in the patent described above is unable to utilize needle mounting by a taper fit described above. The reason is that fitting the needle mounting part to the front-end-part of the outer-cylinder by the taper described above will make it difficult to restrict the retaining position of the based end side of the needle mounting part. Since this type of injector is configured such that the base-end-side of the needle mounting part (protruding part) is engaged with a blind hole at the front-end-part of the inner cylinder thereby allowing the needle to be retracted into the outer cylinder, the retaining position of the base-end-side of the needle mounting part at the opening front-end-part of the injector outer-cylinder is important.

To be more specific, depending on the position of the protruding part in the outer cylinder, the above described engagement is affected. When the retaining position of the needle mounting part (at the opening front-end-part of the injector outer-cylinder) is closer to the front-end-side of the injection needle, and the base-end-side of the needle mounting part does not engage in the blind hole when the inner cylinder front-end is pushed deep into the outer cylinder, the needle will not be retracted into the outer cylinder even if the inner cylinder is pushed back to the front. Therefore, in this retractable injector, the front-end-part of the outer cylinder is not formed in a taper, but formed with a rib on its outer surface so that the position restriction and the water-tightness of the needle mounting part are achieved (however, as described above, a sufficient fitting was not achieved by the above described configuration).

From the above described reason, a general purpose injection needle (hereinafter, also referred to as a general purpose needle) formed for a taper fit can not substantially be mounted on this retractable injector. Although there will be no problem in mounting a special purpose injection needle (a injection needle used for applications in which the needle is retracted into the outer cylinder after use, and hereinafter also referred to as a special purpose needle) on this injector; however, since the injector was often used by replacing the needle, it was a problem that a general purpose injection needle can not be mounted. For example, when resolving a medication, etc. in a vial to inject it into patient's body, the first injection needle to be mounted would be used only for suction and discharge of the solvent, and therefore an inexpensive, general purpose needle with a large gauge number will be sufficient. However, if a general purpose needle can not be mounted on the injector, an expensive, special purpose needle must be used even when its function is not utilized. Therefore, a retractable injector which is unable to be mounted with injection needles other than special purpose needles was not desirable as a matter of practicality.

### Disclosure of the Invention

The above described problems have been successfully solved by proving an invention having following configuration requirements.

The first of the present invention is a safety injector of a type of preventing inadvertent needling by retracting the needle tube part of the injection needle into the outer cylinder after use, characterized in that said safety injector comprises a cylindrical luer part formed in a tip-convergent taper shape at the opening front-end-part of the outer cylinder and an annular protruding part concentrically surrounding the luer part.

The second of the present invention is a safety injector characterized in that an engaging projection part is formed in the front-end-side inner face of the above described annular protrusion.

The third of the present invention is a medical injection needle for use in a safety injector of a type of preventing inadvertent needling by retracting the needle tube part of the injection needle into the outer cylinder after use, characterized by comprising an injection needle, a needle mounting part fixed at the base-end-part of the needle tube part of the injection needle, and a needle base for covering the outside of the needle mounting part excepting the base-end-side of the needle mounting part and retaining the needle mounting part in abutment such that the needle mounting part is detachable in the internal cavity thereof, wherein the shape of the base-end-side of the needle base is arranged so as to be inserted and fitted into the annular gap formed of a cylindrical luer part of the above described first or second injector and an annular projection concentrically surrounding the luer part.

The fourth of the present invention is an injection needle characterized in that in the above-mentioned third injection needle, the base-end-part of the needle base is formed with an engagement projecting part which is engageable with the projection part formed in the front-end-side inner face of the annular protrusion of the above described second injector.

The fifth of the present invention is a safety injector with an injection needle wherein the third or the forth injection needle is mounted on the first or the second injector.

In mounting the third or the fourth injection needle onto the first or the second injector, the mounting is locked by inserting and fitting the base-end-part of the needle base into the annular gap formed of the annular protrusion of the above described injector and the above described cylindrical luer part. Further, upon mounting the fourth injection needle onto the injector of the second invention, the mounting is also locked not only by the above described insertion and fitting, but also by the engagement between the engagement projecting part formed on the base-end-part periphery of the needle base and the engagement projecting part formed in the front-end-side inner surface of the annular protrusion of the injector. By this arrangement, the needle base is more securely locked without falling out into the opening front-end-part. Moreover by adopting such locking method, it is made possible to restrict the position of the needle tube part and the blade face (direction of the blade tip with an acute angle) with respect to the injector. For example, when it is desired to restrict the blade face direction of the injection needle with respect to the marking printed on the outer cylinder of the injector, it was difficult to realize that by a conventional lock-type injection needle or injector, which is formed with a screw, since the blade face is varied by screwing; however, that is realized easily by the locking method of the present invention. Moreover, it also becomes possible to restrict the retention position of the needle mounting part in the injector.

The sixth of the present invention is a safety injector mounted with the above described fifth injection needle, characterized in that both the projection part formed in the base-end-side of the needle base and the projection part formed in the front-end-side inner face of the annular protrusion of the injector are annular.

Further, by forming the cylindrical luer part to be a tip-convergent taper, it is made possible to use not only special purpose needles for safety injectors but also general purpose injection needles by fitting them securely and liquid-tightly into the cylindrical luer part.

The seventh of the present invention is a medical injector in which when the projection part formed in the base-end-side of the needle base and the projection part formed in the front-end-side inner face of the annular protrusion of the injector are engaged, at least a part of the protruding part provided in the base-end-side of the needle mounting part is extended out into the outer cylinder by a predetermined length and the extended length is sufficiently large (so that when the inner cylinder front-end-part is jointed to the outer cylinder internal cavity front-end-part, the above described protruding part is engaged into the blind hole formed in the inner cylinder front-end-part).

By the above described configuration, it is made possible to securely engage the needle mounting part into the blind hole of the plunger thus retracting the injection needle into the outer cylinder when the inner cylinder is pushed into the outer cylinder after use.

Each of the above described inventions can achieve the objects of the present invention at a higher level by adopting the embodiments described below.

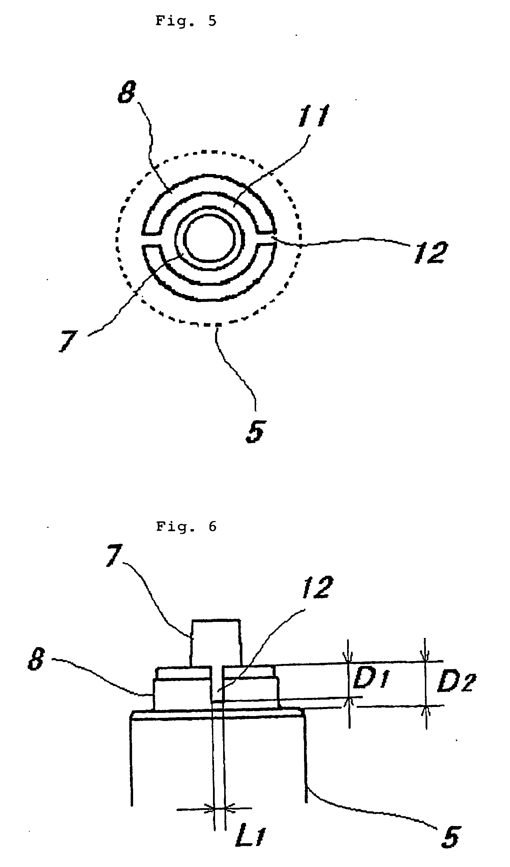

In the above described first and second safety injectors, the annular protrusion is preferably provided with a notch in the axial direction of the outer cylinder. Such notch may include one as shown in Fig. 5 and Fig. 6 in which the annular protrusion is formed with a notch 12 in the direction of the needle axis in such a way as to disconnect the annular shape at the front end of the annular protrusion 8. By providing the notch 12, it is made easy to insert the needle base periphery of the injection needle into the annular gap formed in the front-end-part of the injector. The reason why the insertion becomes easy is speculated that forming the notch 12 makes the annular protrusion 8 to be readily expanded in the radial direction. Therefore, it is important to design the shape, forming position, dimensions, etc. of the notch in such a way that when the annular gap is inserted with a piece having a diameter slightly larger than the internal cavity of the annular projection part, the piece will be received with a cushion. For example, as shown in Fig. 5 and Fig. 6, it is preferable to provide a notch 12 in the direction of needle axis which has a depth D1 of not smaller than 1/3 of the axial length D2 of the annular protrusion 8. The notch 12 may be formed at one position, preferably at 2 or more positions, and more preferably at symmetric positions with respect to the needle axis.

The circumferential length L1 of the notch 12 may be 0.5 to 1.0 mm and the depth D1 in the needle axis direction may be approximately the same as the abutting depth when the needle base is fitted to the annular protrusion.

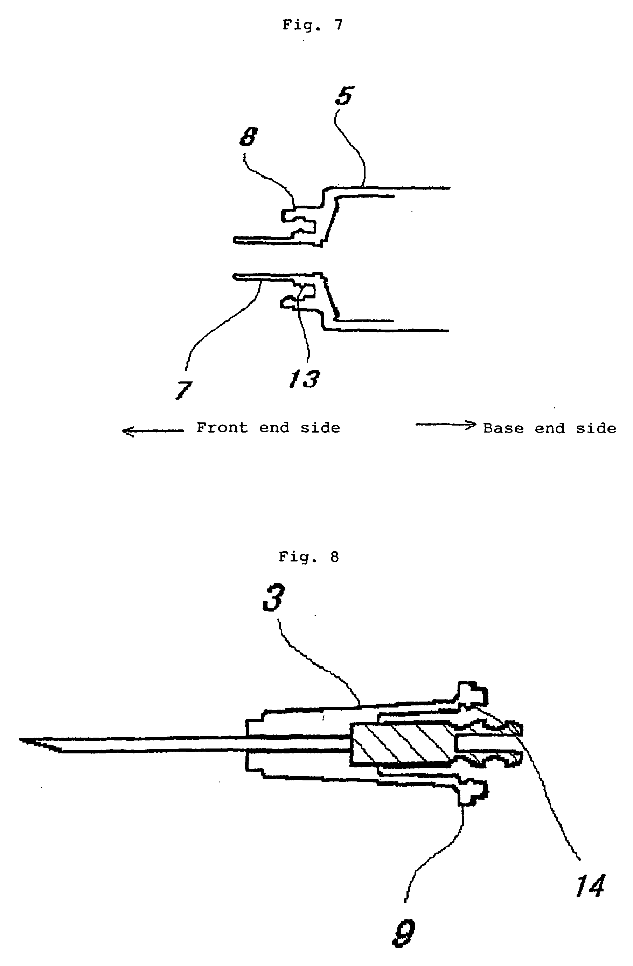

The base-end-side outer face of the cylindrical luer part is preferably formed with an annular projection part for liquid-tight retention. Providing the aforementioned annular projection for liquid-tight retention makes it possible to ensure an enhanced liquid-tightness in particular when a special purpose needle is mounted onto the injector of the present invention as a needle for a safety injector. The above described annular projection part for liquid-tight retention is configured, for example as shown in Fig. 7, by forming an annular projection 13 for liquid-tight retention in the base-end-side outer surface of the cylindrical luer part.

The liquid-tight retention in the case that a special purpose needle is fitted to the opening front-end-part of the injector may be provided by forming an annular projection 14 for liquid-tight retention in the base-end-part internal cavity of the needle base 3 of the injection needle as shown in Fig. 8 instead of the above described annular projection 13 for liquid-tight retention.

Adopting the configuration as described above will result in an enhanced liquid-tightness in the injector due to the fact that the annular projection 13 for liquid-tight retention in the base-end-side outer surface of the cylindrical luer part 7, or the annular projection 14 for liquid-tight retention in the base-end-part internal cavity of the needle base abuts against the internal cavity surface of the needle base or the base-end-side outer face of the cylindrical luer part when a special purpose needle is fitted to the opening front-end-part of the injector.

In the above described third or fourth injection needle, an injection needle in which an engaging projection 9 is formed in an annular shape around the needle axis as shown in Fig. 8 will be fitted more securely when inserted into the annular gap formed in the opening front-end-part of the injector, and will become less vulnerable to detachment and therefore preferable.

However, the engaging projection 9 does not necessarily need to be formed in an annular shape around the needle axis, and may be a single or plurality of independent projections. Moreover, the projection part 10, which is formed in an annular shape around the needle axis, also does not necessarily need to be formed in a continuous annular shape, and may be formed in a partly discontinuous shape.

Moreover, the liquid-tightness of the injector will be substantially improved by the annular projection 14 for liquid-tight retention formed in the base-end-part inner face of the needle base 3.

Hereinafter, preferred embodiments of the present invention will be described with reference to the drawings.

However, the embodiments shown below are intended for showing the preferred embodiments of the present invention and therefore the present invention will not be limited by these embodiments.

### Embodiment 1

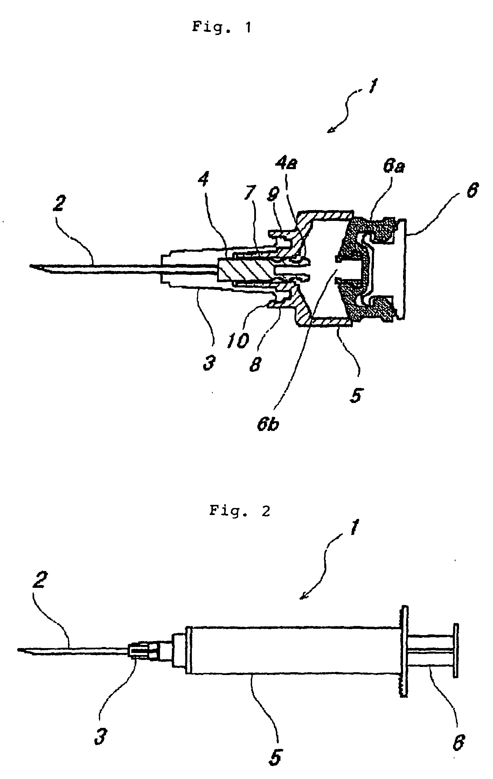

One embodiment of a retractable safety injector mounted with the injection needle of the present invention is shown in Fig. 2. The injector 1 generally consists of a needle tube part 2, a needle base 3, a needle mounting part 4 (not shown), an outer cylinder 5, an inner cylinder 6 (piston member) which is liquid-tightly slidable in the outer cylinder.

An annular protrusion 8 and a cylindrical luer part, which are provided in the front-end-part of the injector and are one of the characteristic features of the present invention, are comprised of flexible plastic such as polypropylene and polycarbonate, and generally molded in one piece, though may be fabricated in any other forming method.

### Embodiment 2

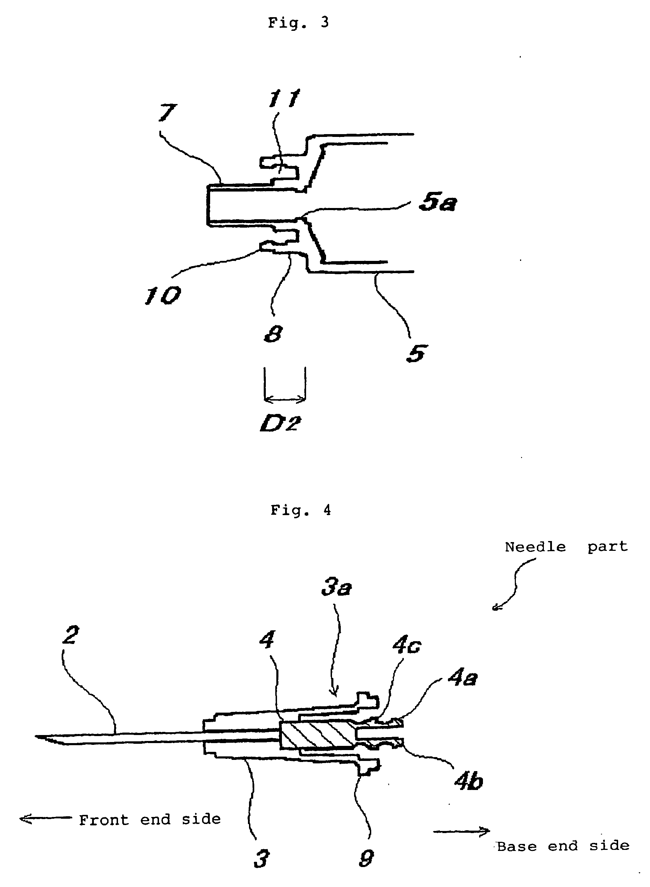

The structure of the opening front-end-part of the outer cylinder of the safety injector is shown in Fig. 3.

The outer cylinder 5 is formed in the opening front-end-part with a cylindrical luer part 7. There is an annular protrusion 8 formed concentrically surrounding the luer part 7. The cylindrical luer part 7 is formed in a tip-convergent taper shape in which the outer diameter of the luer part decreases toward the front end and, more specifically, is preferably formed in a shape with which the luer part can be abutted against and liquid-tightly fitted to the internal cavity of a general purpose injection needle formed with a taper. There is formed an annular gap 11 between the annular protrusion 8 and the cylindrical luer part 7. An edge part 5a is provided in the front-end-side internal cavity of the outer cylinder 5.

The edge part 5a has a function of preventing the retraction of the needle and the needle mounting part into the outer cylinder before use.

### Embodiment 3

One example of the structure of the injection neddle is shown in Fig. 4.

The needle mounting part 4 to which the needle tube part 2 is fixed is retained in abutment against the internal cavity of the needle base 3. The retention of the needle mounting part 4 and the needle base 3 by abutment is arranged such that the needle mounting part 4 is retained by a level of a fitting force whereby the needle tube part 2 and the needle mounting part 4 will be detached from the needle base 3 and be moved when the needle mounting part 4 is pulled in the base-end-side direction by an external force larger than a predetermined level (that is, the engaging force between the inner cylinder front-end and the base-end-side of the needle mounting part as will be described below). The needle mounting part 4 is retained in the needle base in a state that a part of the based end part (protruding part 4a, 4b) is uncovered. There is formed, on the based end side of the needle mounting part 4, a pair of protruding parts 4a, 4b having a shape to engage with a blind hole 6b formed in a plunger 6a at the front-end-part of the inner cylinder 6 thereby allowing the retraction of the needle mounting part 4 into the outer cylinder 5. Abutments 4c is formed in the needle mounting part 4 so that the needle tube part 2 will not be easily moved even when it is pushed toward the outer cylinder 5. Conversely, an annular engaging projection 9 shown in Fig. 4 is formed in the base-end-side peripheral part of the needle base 3 so that the needle base 3 will not be detached from the outer cylinder front-end when the needle mounting part is pushed toward the needle front-end-side.

### Brief Description of the Drawings

Fig. 1 is a schematic diagram to showa fitting state between the injection needle and the outer-cylinder front-end opening of the injector of the present invention. Fig. 2 is a schematic diagram to show the general structure of the injector and the injection needle of the present invention. Fig. 3 is an enlarged sectional view to show the front-end opening of the injector outer cylinder of Fig. 1. Fig. 4 is an enlarged sectional view of the injection needle of Fig. 1. Fig. 5 is a schematic diagram to show a state that the front-end-part of the outer cylinder of Fig. 6 is sectioned. Fig. 6 is a schematic diagram viewed from side to show the outer-cylinder front-end-part of the medical injector of the present invention. Fig. 7 is an enlarged sectional view to show the outer-cylinder front-end opening of the injector provided with an annular projection for liquid-tight retention. Fig. 8 is an enlarged sectional view to show the injection needle of the present invention in which an annular projection for liquid-tight retention is provided in the base-end-part internal cavity of the needle base of the injection needle.

### Best Mode for Carrying out the Invention

The base-end-side peripheral part 3a of the needle base 3 shown in Fig. 4 is inserted and fitted into the annular gap 11 formed between the annular protrusion 8 of the opening front-end-part of the outer cylinder 5 shown in Fig. 3 and the cylindrical luer part 7. At this moment, designing the inner diameter of the annular protrusion 8 to be equal to or slightly smaller than the outer diameter of the engaging projection 9 of the needle base 3 will increase the fitting strength and make the needle base hard to be detached once it has been fitted. With dimensions of the latter design, it will become difficult to insert the needle base peripheral part into the annular gap upon fitting. However, since the annular protrusion 8 deflects outwardly due to a notch 12 formed on the annular protrusion 8 thereby slightly expanding its inner diameter, the needle base peripheral part 3a will be inserted into the annular gap 11 with relatively ease.

As shown in Fig. 3, on the front-end-side inner face of the annular protrusion 8, there is formed an annular engaging projection part 10 for firmly fitting the needle base 3 into the opening front-end-part of the injector in cooperation with the engaging projection 9. Owing to the engaging projection part 10, the needle base peripheral part 3a fitted into the gap 11 is prevented from being detached with an outer force of a normally anticipated level. The fitting state by this engaging projection part 10 is shown in Fig. 1. It is desirable to form at least one of the engaging projection 9 and the engaging projection part 10 in an annular shape. Although forming neither one of them in an annular shape will require alignment to engage both of them, forming either of them in an annular shape will eliminate the time and effort for the alignment. In the present embodiment, both the engaging projection 9 and the engaging projection part 10 are formed in an annular shape thereby increasing the fitting strength, which is a more preferable embodiment.

In the case of mounting a special purpose needle for needle retraction onto the injector 1 of the present embodiment, since the engagement between the engaging projection 9 and the engaging projection part 10 or, in other words, the fitting of the needle base peripheral part into the annular gap 11 is intended for the enhancement of the fitting strength and the positional restriction of the protruding parts 4a, 4b at the base-end-side of the needle mounting part in the outer cylinder, the liquid-tight retention is not so sufficient as will be obtained by a typical taper fit (in the case of a general purpose needle). Therefore, to achieve a higher liquid-tightness, it is preferable to provide an annular projection 13 for liquid-tight retention in the base-end-side outer face of the cylindrical luer part as shown in Fig. 7, or an annular projection 14 for liquid-tight retention in the base-end-part internal cavity of the needle base 3 as shown in Fig. 8.

Since the injection needle is to be retracted into the outer cylinder (a) after finishing the injection of the medication in the injector, it is required that the base-end-side (protruding part) of the needle mounting part which is mounted in the internal cavity of the needle base extends out into the outer cylinder by a predetermined length when the engaging projection 9 of the needle base of a special purpose needle is engaged with the engaging projection part 10 formed in the annular protrusion part. That is, it is required that upon abutting the inner cylinder front-end-part against the front-end-part of the outer-cylinder internal cavity (that is, upon abutting the inner cylinder into the deepest part of the outer cylinder after finishing the injection of the medication), the protruding part of the needle mounting part must extend out by a length so as to be engaged into the blind hole formed at the inner-cylinder front end. Though, controllable (adjustable) dimensions for that purpose include (1) the length of the protruding part 4a, 4b formed in the needle mounting part, (2) the depth of the blind hole 6b, (3) the axial length D2 of the annular protrusion 8, (4) the forming position (in the axial direction) of the engaging projection part, (5) the width/forming position of the engaging projection 9 of the needle base, it is preferable to control the latter three which are more easily adjustable. Since the former two affect the remaining amount of the medication in the injector, it is difficult to significantly vary them. Therefore, in practice, an extension length of the protruding part 4a, 4b which can be extended out into the outer cylinder is determined and thereby the depth of the blind hole 6b is to be specified. It is also possible to form an engaging projection 9 of the needle base and an engaging projection part 10 formed on the annular protrusion in such a way that the tip of the protruding part 4a, 4b will be positioned at a predetermined extension length in the outer cylinder by pushing in the inner cylinder after use. For example, the axial depth of the annular protrusion 8, the forming position (in the axial direction) of the engaging projection part, and the width/forming position of the engaging projection of the needle base may be determined.

### Industrial Applicability

The present invention has successfully provided an injection needle and injector for a safety injector of a type of preventing inadvertent needling by retracting the needle tube part of the injection needle into the outer cylinder after use, wherein the problem of detachment of the needle and the needle base from the injector is solved, and not only special purpose injection needles for the safety injector but also general purpose injection needles can be used, and particularly an injection needle and injector having an improved fitting structure.

## Claims

1. A safety injector of a type of preventing inadvertent needling by retracting the needle tube part of the injection needle into the outer cylinder after use, **characterized in that** said safety injector comprises: a cylindrical luer part formed in a tip-convergent taper shape at the opening front-end-part of the outer cylinder; and an annular protrusion concentrically surrounding said luer part.

2. The safety injector according to claim 1, **characterized in that** an engaging projection part is formed in the front-end-side inner face of said annular protrusion.

3. The safety injector according to claim 1 or 2, **characterized in that** said annular protrusion is formed with a notch in such a way as to disconnect the annular shape.

4. The safety injector according to claim 1, 2, or 3, **characterized in that** the base-end-side outer face of said cylindrical luer part is formed with an annular projection for liquid-tight retention.

5. A medical injection needle for use in a safety injector of a type of preventing inadvertent needling by retracting the needle into the outer cylinder after use, **characterized by** comprising an injection needle part, a needle mounting part fixed at the base-end-part of the needle tube part of said injection needle part, and a needle base for covering the outside of the needle mounting part excepting the base-end-side of the needle mounting part to abut and retain the needle mounting part so as to be detachable in the internal cavity, wherein the base-end-part periphery of said needle base is formed with an engaging projection part.

6. The medical injection needle according to claim 5, **characterized in that** said engaging projection part is formed in an annular shape around the needle axis.

7. The medical injection needle according to claim 5 or 6, **characterized in that** an annular projection for liquid-tight retention is formed in the internal cavity at the base-end-part of said needle base.

8. A safety injector with an injection needle, **characterized in that** the injection needle according to claim 5, 6, or 7 is mounted on the injector according to claim 1, 2, 3, or 4.

9. The safety injector with an injection needle according to claim 8, **characterized in that** the engaging projection of the base-end-part periphery of said needle base and the engaging projection part at the front-end-side inner face of the annular protrusion of said injector are engaged with each other.

10. The medical injector according to claim 8, wherein when the engagement projection on the base-end-part periphery of said needle base and the engagement projection on the front-end-side inner face of the annular protrusion of said injector are engaged with each other, at least a part of the protruding part provided in the base-end-side of the needle mounting part is extended out into the outer cylinder by a predetermined length, and the extended length is sufficiently large so that when the inner cylinder front-end-part is jointed to the internal cavity front-end-part of the outer cylinder, said extended protruding part is engaged into a blind hole formed in the inner cylinder front-end-part.

11. The safety injector with an injection needle according to claim 8, 9, or 10, **characterized in that** the engaging projection formed at the base-end-part periphery of said needle base is inserted into the annular gap formed of said cylindrical luer part formed in the front-end-part of the outer cylinder of said injector and said annular protrusion, and said engaging projection is engaged with the engaging projection part formed in the front-end-side inner face of said annular protrusion.
